(19) **Europäisches Patentamt European Patent Office Office européen des brevets**

(11) **EP 4 246 394 A1**

(12) **EUROPEAN PATENT APPLICATION**

(43) Date of publication:
**20.09.2023  Bulletin 2023/38**

(21) Application number: **22161968.7**

(22) Date of filing: **14.03.2022**

(51) International Patent Classification (IPC):
**G06Q 10/06** (2023.01)    **G09B 19/00** (2006.01)
**G09B 5/06** (2006.01)    G06Q 50/04 (2012.01)
**G09B 21/00** (2006.01)

(52) Cooperative Patent Classification (CPC):
**G06Q 10/06398; G06Q 10/063114;**
**G06Q 10/06316; G09B 5/06; G09B 19/003;**
G06Q 50/04; G09B 21/006; G09B 21/008

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koa Health B.V. Sucursal en España**
**08018 Barcelona (ES)**

(72) Inventors:
• **Garcia i Tormo, Albert**
**08018 Barcelona (ES)**

• **Hemmings, Nicola**
**08018 Barcelona (ES)**
• **Vowell, Claire**
**08018 Barcelona (ES)**
• **Buda, Teodora Sandra**
**08018 Barcelona (ES)**
• **Vermeulen, Remko**
**08018 Barcelona (ES)**

(74) Representative: **Herrmann, Uwe**
**Lorenz Seidler Gossel**
**Rechtsanwälte Patentanwälte**
**Partnerschaft mbB**
**Widenmayerstraße 23**
**80538 München (DE)**

(54) **ASSESSING USER ENGAGEMENT TO IMPROVE THE EFFICACY OF MACHINE-USER INTERACTION**

(57)    The present invention relates to a device that based on a model automatically tailors the communication mode with a user to the individual characteristics of the user without the user having to provide active input regarding their preferences. Instead, the model selects the most suitable motivator to ensure the user is engaged in performing an instructed activity, e.g. a step of controlling the machine in a complex manufacturing process. Thus, conscious biases of the user, such as the user stating to prefer written text but in fact being more responsive to videos, do not hamper the interaction of the device with the user and the efficacy of machine-user interaction is improved.

**Description**

In the modern world due to an increasing digitalization and automation, ensuring an efficient interaction of human users with a plurality of devices, such as machines or electronic devices, becomes increasingly important. In this context, ensuring user engagement has emerged as one of the main challenges in ensuring an efficient and stable machine-user interaction.

In practice, often users do not feel engaged and feel reluctant to interact with a device or eventually even stop interacting with the device altogether.

Previous attempts to improve user engagement have generally focused on designing user friendly application interfaces or on adjusting the content delivered. For example, if the user repeatedly fails to perform maintenance work on the device after the device has issued a corresponding alert, the device may issue an instruction to contact another maintenance worker, based on the assumption that the user might be capable of performing this simpler activity.

However, with these previous solutions focus only on adjusting the contents (this is, the "what") to maximise user engagement; with conventional devices delivering the same content in the same way for all users, regardless of the characteristics of the individual user. Individual users, however, may differ significantly from each other and may respond to different modes of interaction differently.

In an analogy with sports, the activity could be improving the punching technique in martial arts. Some students may learn well by carefully observing the instructor (focusing on the position of the legs and movement of the hips, shoulders and arms) and practising on their own; other students may need to practise with the punching bag to feel the strength when properly coordinating the movement; other students may need the instructor or a colleague to help them positioning themselves at the right intermediate positions.

In all cases the "what" is the same (improving the punching technique), but the "how", the way of engaging in the activity, of learning or achieving the improvement, may differ. Probably all students could learn with all methods, but, for each individual, there is one (or a few) method(s) that allow them to learn faster and/or to learn further than with the others. Likewise, it may be needed to change the method depending on the moment, in order to keep improving. The challenge is, for each student and every moment in time, to find out what is the most effective method to improve the punching technique.

Customising the way in which the content is delivered (this is, the "how"), however, remains typically unexplored in the context of human-machine-interaction and it unfolds different ways for addressing user engagement. Thus, there exists a need to provide a device of interacting with an individual user to induce that particular user to interact with the device in the desired way. Following up on the example given above, there exists a need for a device capable of interacting with the user to the effect that the user actually successfully performs the required maintenance work and not only the simpler task of calling another maintenance worker.

It is the object of the present invention to alleviate or even completely overcome the problems of the prior art. This object is achieved by the subject-matter of the independent claims. Advantageous embodiments of the invention are the subject-matter of the dependent claims.

A device according to the present invention for assessing and / or improving the engagement of a user in at least one activity is configured to:

- Instruct the user to perform at least one activity by means of an output unit,
- Monitor at least one first parameter by means of a monitoring unit to assess the engagement of the user in performing the at least one activity or attempting to perform the at least one activity,
- Expose the user to at least one first motivator by means of the output unit, wherein the motivator is chosen to prompt or motivate the user to perform the at least one activity or to attempt to perform the at least one activity,
- Monitor at least one second parameter by means of the monitoring unit to assess the engagement of the user in the at least one motivator,
- Enter the first and second parameters into a model of the user that indicates the efficacy of at least one motivator for motivating the user to perform or to attempt to perform at least one activity, wherein the model is preferably customized to the individual user,
- Compare by means of a comparison unit the at least one first parameter or an activity engagement score based on the at least one first parameter to a threshold value, and / or
- Compare by means of the comparison unit the at least one second parameter or a motivator engagement score based on the at least one second parameter to a threshold value,
- If the comparison indicates that the at least one first parameter or the activity engagement score and / or the at least one second parameter or the motivator engagement score is below the threshold value,
- Select by means of the model at least one second motivator from a list of motivators accessible by the model, and
- By means of a control unit control the device to expose the user to the least one second motivator by means of the output unit.

For example, the device is a machine used in manufacturing and the user is required to interact with the machine with a high degree of engagement to control the machine during a complex manufacturing process. Initially, the machine may issue an instruction to the user via an output unit to instruct the user to walk to a storage room and fetch a required component.

For example by means of a wearable device comprising accelerometer data it can be assessed how much time the user spends walking. This gives an indication of the time the user performs the instructed activity or at least attempts to do so.

To motivate the user to successfully perform the instructed activity, the machine exposes the user to a motivator, for example, a text appearing on a screen of the machine explaining why the component to be fetched is important for the subsequent manufacturing process.

The motivator may be issued simultaneously with the instruction to perform the activity or with a delay. The engagement of the user with the motivator is assessed, e.g. by measuring the time the user spends reading the text.

The data indicating the engagement of the user in the activity, in this example the time the user spent walking, and the data indicating the engagement of the user with the motivator, in this example the time the user spent reading the text, are then input into a model of the user that for example indicates that this particular user is reasonable responsive to motivators in the form of text but is much more responsive to motivators in the form of videos.

It is then assessed whether the engagement of the user in the activity is satisfactory. For example, the time the user spent walking (e.g. 2 min) is compared to the time it should take the user to fetch the component from the storage room (e.g. 5 min). The measured walking time may be compared directly to a threshold value, for example, the measured 2 min of walking may be compared to 4 min corresponding to the time even a very fast walker would require to fetch the component. Alternatively, a quotient may be calculated, for example 2 min measured activity out of 5 min required activity, corresponding to a quotient of 2/5= 0,4. This quotient 0,4 may then be compared to a threshold value of 2/4= 0,5 indicating a very fast walker successfully completing the activity.

The time the user spent engaged with the motivator, i.e. the time the user spent reading the text may alternatively or additionally similarly be compared directly to a threshold value or a score, such as a quotient may be calculated that is then compared to a threshold value.

If the comparison with the threshold value indicates that the user did not perform the activity satisfactorily, e.g. the user did not walk far enough, and / or the user did not engage satisfactorily with the motivator, e.g. the user read the text only for 30 sec although 1 min would be required for attentive reading, the model is used to select at least one different motivator, for example a video illustrating the instructed activity or a video explaining why the required component is important. For example, in the past, the user has shown to be responsive to videos.

In this case, the model selects a video as an alternative or additional motivator. The control unit of the machine then controls the machine to display the video selected by the model to the user.

Thus, preferably a device according to the present invention tailors the communication mode with a user to the individual characteristics of the user without the user having to provide active input regarding their preferences. Instead, the model selects the most suitable motivator. Thus, conscious biases of the user, such as the user stating to prefer written text but in fact being more responsive to videos, do not hamper the interaction of the device with the user. In other words, a device according to the invention preferably phrases instructions to the user (e.g. by choosing suitable motivators) in a way that the user is optimally responsive.

The example given above is only an example and the present invention may be also used for other exemplary applications, for example, to encourage an employee to follow an online course on cyber security or another topic or attentively follow a manual or video detailing how to operate a given machine. Also, in order to ensure safety of the user, the user may be encouraged to take e.g. three breaks from work each day and spend these breaks walking to increase productivity and concentration of the user, e.g. if the user has to guide a machine through a complex process over several hours. Generally, the tasks or instructed activities may be set by the user themselves (the user thus performs the instructed tasks voluntarily) and / or by another person and / or device.

According to an embodiment of the invention, the device is configured to keep selecting another motivator until the comparison indicates that the at least one first parameter or the activity engagement score and / or the at least one second parameter or the motivator engagement score meets or surpasses the threshold value. In other words, the device may be configured to prompt the user with different motivators until a desired level of engagement of the user in an instructed activity and / or with a motivator has been achieved.

If it is detected by the device that the at least one first parameter or the activity engagement score and / or the at least one second parameter or the motivator engagement score fails to meet or surpass the threshold value for a defined period of time, the device may be configured to notify an external source, e.g. an external device, accordingly. The device may issue a notification to another device that may be of a higher instance or person, for example a device or person that set the instructed activity.

This way, preferably, in cases in that the user is highly engaged but fails to complete the task in the defined period of time or in that user engagement can not be achieved to a satisfactory level by the device in the defined period of time, a notification or report is issued to a higher level.

The defined period of time may be set by default, by the person or device setting the instructed activity or may be automatically set by the device, preferably in an individualized way for each user instructed to perform a task.

According to an embodiment of the invention, the threshold is predefined, a default value or determined for each individual user.

Of course, the device may be configured to instruct multiple activities and enforce these activities each with a different motivator that is particularly suitable for increasing user engagement with that activity.

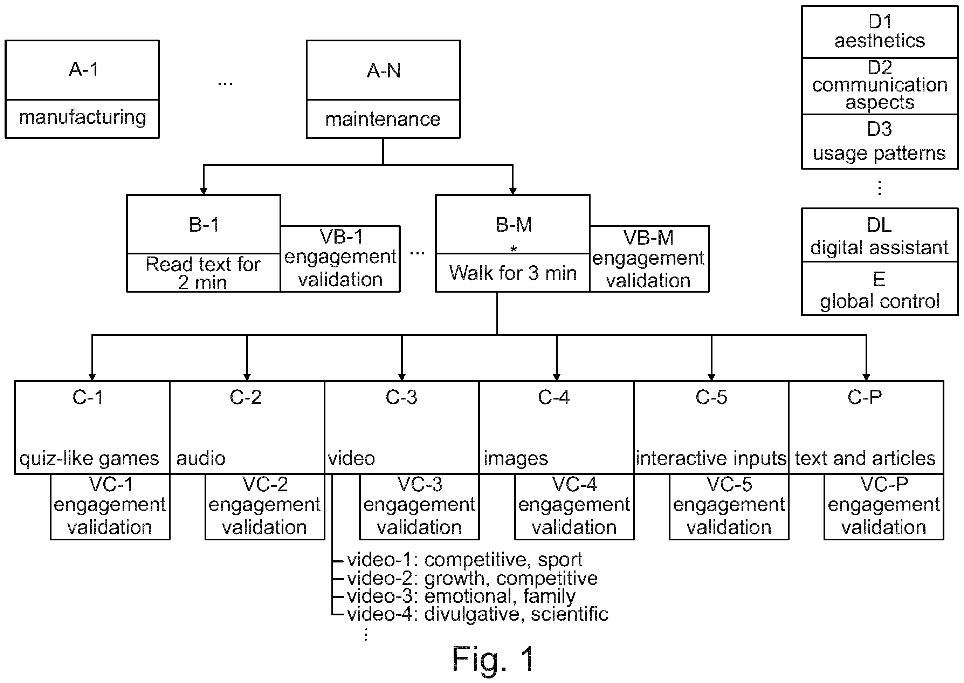

According to an embodiment of the invention, the model comprises a plurality of task elements (for example, the elements A in the exemplary model shown in Fig. 1). Each task element is associated with a plurality of activity elements (for example, the elements B in the exemplary model shown in Fig. 1) that preferably correspond to different activities that the user may be instructed to perform in the course of completing the task.

According to an embodiment of the invention, the model comprises a plurality of activity elements (for example, the elements B in the exemplary model shown in Fig. 1) each comprising a designated validation element (for example, the elements VB in the exemplary model shown in Fig. 1) configured to assess the engagement of the user in the activity corresponding to the activity element.

Preferably, the model further comprises for each activity element a plurality of motivation elements (for example, the elements C in the exemplary model shown in Fig. 1) each comprising a designated validation element (for example, the elements VC in the exemplary model shown in Fig. 1) configured to assess the engagement of the user in the motivation element corresponding to the activity element. Preferably, a motivation element may also be regarded as a motivator.

Further preferably, the model further comprises for each motivation element a plurality of motivation specimens, wherein the motivation element preferably defines a group into that all the motivation specimens associated with this motivation element fall. For example in the model shown in Fig. 1 videos video-1 to video-4 are motivation specimens associated with the motivation element C-3 defining the group "videos".

According to an embodiment of the invention, the model is continuously updated based on parameters monitored by the monitoring unit, such as the first and second parameters entered into the model, and / or calculated values derived from these parameters. This has the benefit that the model constantly learns about changing characteristics and preferences of the user thereby allowing for a constantly accurate and up-to-date selection of the most suitable motivator(s).

Preferably, the model is automatically updated without any specific input to that effect from the user. Generally, a device according to the present invention preferably automatically analyses the interaction of the user with the device and / or the behaviour of the user and / or passively acquires data relating to the user and thus is capable of optimizing the communication of the device with the user without the user having to actively provide input to that effect.

Further, the device may be configured to, by means of an evaluation unit, evaluate for a past time interval a change in the at least one first parameter or the activity engagement score and / or the at least one second parameter or the motivator engagement score.

The past time interval may be for example, the last hour, the last day, the last week, the last year or any other past time interval. The time interval may also span the time between two attempts of the user of completing the instructed activity and / or between two instances of interaction of the user with the device. For example, it may be evaluated how the engagement of the user in the instructed activity and / or the engagement of the user with the motivator may change with time.

According to an embodiment, a device according to the invention is further configured to based on parameters monitored by the monitoring unit, such as the first and second parameters entered into the model, and / or calculated values derived from these parameters, identify by means of the model at least one attribute of the user that characterizes the user and in particular indicates a preference of the user for at least one motivator.

For example, it may be measured that the user often clicks on a "learn-more"- button present on a graphical user interface of the device to learn more about an instructed activity. From this, the model can derive that the user is relatively curious and / or that the user performs activities more reliably if they understand the reason for the activity. Thus, the model may select as a motivator to expose the user to interesting background information about an instructed activity to induce the user to more reliably perform the instructed activity.

According to another embodiment, a device according to the invention may be configured to by means of the model, select from a stored list of support sources based on the at least one attribute of the user a support source expected to optimally support the user in performing the at least one activity or attempting to perform the at least one activity, wherein the support source preferably is person or a virtual support system, such as a chat bot or an avatar.

For example, if the model has determined that the user is relatively sociable, the model may select a friendly avatar to be displayed by the device on a graphical user interface to the user to guide the user through the steps of e.g. a complex manufacturing process to be performed by the device. If the model, however, has for example determined that based on the user's age and behaviour, the user is likely to be more comfortable with a book than with a graphical user interface, the device may draw the user's attention to a physical manual.

When selecting a support source the device may draw on a data base of possible support sources.

In case of virtual support systems, such as chat bots or avatars, the device may retrieve a suitable chat bot or avatar from a database of chat bots and avatars.

In order to further improve user engagement, the device can be configured to generate based on the at least one attribute of the user a virtual support system expected, e.g. based on the model, to optimally support the user in performing the at least one activity or attempting to perform the at least one activity, wherein the virtual support system is a chat bot or an avatar.

In other words, if no optimally suitable chat bot or avatar for a particular user can be retrieved from the database, the device can generate a customized virtual support system, such as a chat bot or an avatar, for the user. For this effect, an existing chat bot or avatar may be modified or a new chat bot or avatar may be generated from scratch.

According to an embodiment of the invention, the device is configured to instruct the user to perform a plurality of activities by means of the output unit, and for each activity of the plurality of activities expose the user to at least one first motivator, wherein the motivators may differ from each other.

For example, the user may be instructed to walk to the storage room to fetch a component and to keep an eye on a temperature sensor. For the first activity, the user may be most responsive to a text providing background information on the importance of the component and for the second activity the user may be most responsive when exposed to a video illustrating the effects of over-heating.

According to an embodiment, different motivators are configured to convey the same content to the user in different formats and / or modes of delivery. For example, one motivator may be a text providing background information why a component is required and a second motivator may be a video providing background information why the component is required.

Another aspect of the invention relates to a method for assessing and / or improving the engagement of a user in at least one activity, the method preferably being performed by a device according to the invention, the method comprising:

- Instructing the user to perform at least one activity, preferably by means of an output unit,
- Monitoring at least one first parameter by means of a monitoring unit to assess the engagement of the user in performing the at least one activity or attempting to perform the at least one activity,
- Exposing the user to at least one first motivator, preferably by means of the output unit, wherein the motivator is chosen to prompt or motivate the user to perform the at least one activity or to attempt to perform the at least one activity,
- Monitoring at least one second parameter, preferably by means of the monitoring unit, to assess the engagement of the user with the at least one motivator,

- Entering the first and second parameters into a model of the user that indicates the efficacy of at least one motivator for motivating the user to perform or to attempt to perform the at least one activity, wherein the model is preferably customized to the individual user,
- Comparing, preferably by means of a comparison unit, the at least one first parameter or an activity engagement score based on the at least one first parameter to a threshold value, and / or
- Comparing, preferably by means of the comparison unit, the at least one second parameter or an motivator engagement score based on the at least one second parameter to a threshold value,
- If the comparison indicates that the at least one first parameter or the activity engagement score and / or the at least one second parameter or the motivator engagement score is below the threshold value, selecting by means of the model at least one second motivator from a list of motivators accessible by the model, and
- Exposing the user, preferably means of a control unit controlling the device, to the least one second motivator, preferably by means of the output unit.

Preferably, the model is continuously updated based on parameters monitored by the monitoring unit, such as the first and second parameters entered into the model, and / or calculated values derived from these parameters.

Preferably, the method further comprises the step evaluating for a past time interval, preferably by means of an evaluation unit, a change in the at least one first parameter or the activity engagement score and / or the at least one second parameter or the motivator engagement score.

According to an embodiment, the method further comprises the step: based on parameters monitored preferably by the monitoring unit, such as the first and second parameters entered into the model, and / or calculated values derived from these parameters, identifying by means of the model at least one attribute of the user that characterizes the user and in particular indicates a preference of the user for at least one motivator.

Preferably, a method according to the inventions is performed automatically, preferably without the user being required to provide specific input beyond the interaction of the user with the device.

According to another embodiment, the method further comprises by means of the model, selecting from a stored list of support sources based on the at least one attribute of the user a support source expected to optimally support the user in performing the at least one activity or attempting to perform the at least one activity, wherein the support source preferably is person or a virtual support system, such as a chat bot or an avatar.

Preferably, a model used in the context of a method according to the invention comprises the same features as a model described in the context of a device according to the invention.

Preferably, a method according to the invention further comprises generating based on the at least one attribute of the user a virtual support system expected to optimally support the user in performing the at least one activity or attempting to perform the at least one activity, wherein the virtual support system is a chat bot or an avatar.

According to another embodiment, the method further comprises instructing the user to perform a plurality of activities by means of the output unit, and for each activity of the plurality of activities exposing the user to at least one first motivator, wherein the motivators may differ from each other.

Preferably, different motivators are configured to convey the same content to the user in different formats and / or modes of delivery.

In other words, the invention may be described as follows:

When a user interacts with a device, it often is necessary to induce the user to perform a specific action or activity. Thus, the device is preferably controlled to induce the user to perform a defined action or activity and ensure satisfactory user engagement, e.g. for the user to follow through with completing the instructed activity. In other words, something is defined that the user must do (i.e. the *what).* However, the way of reaching out to the user, this is, how to make sure that the user understand what they need to do and making them do it and engage in the instructed, is something that can be done in different ways.

For example, some users may engage in a quiz-like game, as a challenging or competitive activity; some other users may dislike the competitive aspect and may prefer to watch a video. Despite using different means for reaching, motivating and involving the user, in both cases, quiz and video, the goal is the same: to induce the user to perform a specific activity or action. In other words, preferably the same instruction (i.e. the same *what)* is delivered in different ways (different *how)* tailored to individual users.

For example, an action or activity can be motivated and/or delivered in multiple ways, including quiz-like games, images, audio, video, interactive inputs and / or texts. Each of the instructions can be hence be delivered in any of these ways, which unfolds a wide range of possibilities of reaching to users and keeping users engaged.

The present invention addresses the need of improving user engagement in the interaction of the user with a device by optimizing the way in which an instruction is delivered in order to maximise its effectiveness. The most effective way of delivering an instruction is not necessarily the one that the user would consciously choose or claim to feel most comfortable with. Thus, finding the most effective way to deliver an instruction to a user (in other words, reaching to the user) and ensuring the user is motivated to perform the instructed action is an issue that has remained unaddressed by prior art solutions.

The present invention offers according to an advantageous embodiment the automatic adjustment of the *how,* the way of delivering the instruction, aiming to maximise the user engagement. Maximising the user engagement preferably does require distinguishing that which is preferred by the user from that which is effective for that user, an aspect that is an key element of an embodiment of the invention.

Adjusting the how (or personalising the delivery of the instruction) involves not only adapting a variety of aspects such as the aesthetics of a graphical user interface presented to the user, the mode or way of interacting with the device (e.g. voice or text, for both input and output) or the amount of contextual information provided by default, but also adapting the format in which the instruction is delivered (e.g. in the form of quiz-like games, via audio output, video, images, interactive inputs or texts).

Likewise, personalising the delivery preferably requires monitoring several aspects of the user, including both behaviour-based aspects and physiological-based aspects. These aspects may be monitored by a monitoring unit that for example includes sensors. The measurements preferably are based on high time-resolution data (inputs that are gathered frequently, from daily activities, sometimes even on the moment), in order to distinguish that which is effective for the user from that which is preferred by the user.

It might be less advantageous to infer such personalisation in a non-automatic way, since gathering daily inputs would require the continuous supervision of the user to assess their behaviour. Even if continuous supervision were feasible, the observed user behaviour would be conditioned by the fact that another person would be supervising the user (biased measurement).

Thus, parameters of the user are preferably automatically monitored by a monitoring unit without the user noticing and / or without the user having to actively provide input. The monitoring unit may comprises several sensors, such as proximity sensors, GPS-location-sensors, gyrometers, a camera, a microphone, accelerometers etc. The monitoring unit may for example comprise portable devices such as a wearable device present on the user.

Further features, elements, advantages and effects of the present invention may become apparent from the following description of exemplary embodiments of the invention referring to the figures. In the figures, the same reference numerals indicate the same or similar elements.

The exemplary embodiments illustrate but not limit the present invention, as the present invention is defined by the claims.

Fig. 1 shows a preferred embodiment of the invention focusing in particular on an embodiment of a model used by the present invention;

Fig. 2 shows an example of engagement evaluation based on an exponential model;

Fig. 3 shows an example of a breathing signal measured with a camera (upper plot) and the inhalations and exhalations identified from the derivative signal (lower plot) by comparing with a threshold;

Table 1 shows an example of evaluating a current user engagement based on an exponential model; and

Table 2 shows exemplary scenarios relating to user engagement in an activity and with a motivator and exemplary actions taken by the device.

A preferred embodiment of the invention, in particular of a model used in the context of the present invention, is shown in Fig. 1 and described in more detail below:

A device according to the invention comprises both hardware and software elements.

A device according to the present invention may be a mobile phone, a computer, a tablet or similar device that may be part of another device, e.g. a manufacturing machine or that may be separate. The device preferably comprises a number of hardware sensors, for example a camera, a microphone, a speaker, a screen, a keyboard, a touchscreen or a mouse. The sensors providing data to the device may be part of a different device, like a smart watch. A device according to the present invention may be configured to additionally access external resources like remote servers.

One aspect of a device according to the present invention is a model indicating for a user which delivery of an instruction (e.g. an instructions supported by a motivating video) leads to optimal user engagement.

Additionally or alternatively, a device according to the present invention preferably comprises at least one speech-to-text and / or a text-to-speech conversion unit and / or a unit configured for object recognition in images. The control unit of the device may be configured to perform the functions of these units. Furthermore, the device preferably is configured to assess a database comprising the materials belonging to the different instructions, activities or motivators such as videos, texts, images, etc.

The provision of a speech-to-text and / or a text-to-speech conversion unit allows the device to interact with a user who is e.g. visually impaired or not capable of reading and / or writing.

The model in this embodiment comprises multiple units (that can also be regarded as blocks or modules) at different levels; the block hierarchy is preferably introduced to advantageously cluster the elements.

The embodiment of the model shown in Fig. 1 comprises multiple engagement validation blocks, which describe the aspects that are to be monitored in order to evaluate the engagement related to that block. The term engagement validation preferably applies to both the engagement in an activity and the engagement in the motivation for an activity.

Another element of the model is the validation number, wherein the term number preferably may involve a complex data structure like a vector of numbers with timestamps, part of the engagement validation blocks, which is evaluated based on the criteria defined by the validation blocks. The validation number may be a score indicating the engagement of the user in an activity and / or with a motivation means or motivator. The model also includes global blocks, which are used to monitor and / or control general aspects such as the aesthetics of a graphical user interface presented to the user (e.g. modifying the size of the buttons), the way of writing (e.g. communication style, e.g. colloquial or very formal communication) or the usage patterns (e.g. usual time of the day for interacting with the device, frequency of the interactions, etc.).

In this example, besides the model, the device further comprises a control unit which preferably analyses the model and / or receives input therefrom, preferably in particular the engagement values captured by the model, and changes the mode of the delivery of an instruction accordingly. For example, the output provided by the device to the user may be changed from video-1 to video-2 to motivate the user to engage in activity B-1 or B-4). The control unit may also connect to external resources, like a databases, to exchange information. The control unit preferably initialises the model when creating it.

The model shown in Fig. 1 summaries the user needs in terms of instruction delivery and it preferably is regularly updated. The goal of this embodiment shown in Fig. 1 is to induce the user to systematically try to complete an instructed activity (can also be referred to as an action or task) and to achieve this different motivation means or motivators are used. When the user is systematically trying to complete the instructed activity, then the engagement is high, regardless of whether the user successfully completes the activity.

As shown in Fig. 1, the model may be organised in blocks, which are grouped in different levels. A first level splits the blocks (e.g. blocks A, ranging from A-1 to A-N, wherein N may be any real, positive number)) according to the overall task or topic, such as manufacturing or maintenance. Each user may be involved in more than one topic at a time; typically up to three topics simultaneously.

Each element A has, as sub-elements, all possible activities or actions that the user may be instructed to perform and that relate to the specific topic corresponding to each block A. In Fig. 1, these sub-elements are labelled blocks B (ranging from B-1 to B-M, wherein M may be any real, positive number). An example of an element B is reading a text for 2 min or walking for 3 min. Each of the elements B preferably involves the user interacting with the device directly or indirectly (an example of indirect interaction is passive sensing or monitoring of parameters, e.g. parameters related to the user, while the user does activities).

In Fig. 1, some of these elements B are marked by an asterisk(*), for example, the activity B-M in Fig. 1. Elements B marked in this way correspond to activities the user should perform. The other ones are simply not applicable for this user this time.

Each of the elements B has an associated validation block (labelled as elements VB in Fig. 1, ranging from VB-1 to VB-M). These validation blocks preferably describe the parameters that have to be monitored in order to determine the user engagement in this activity. An example of a validation block VB could be, for the case of walking 3 min, monitoring the GPS data and the pedometer data (derived from the accelerometer) to objectively determine whether the user has attempted to complete the task and / or with how much effort the user has attempted to complete the task.

The elements B correspond to the activities themselves; each of these elements can be presented to the user or motivated, for example supported or accompanied by at least one motivator, in a different way. Each element B is associated with or includes all possible motivators or motivation means (or ways of motivating the user to engage in this activity). In the example shown in Fig. 1, the motivation means are depicted as elements C ranging from C-1 to C-P (wherein P may be any real positive number) and corresponding to presenting a quiz game, audio, video, images, interactive inputs or texts and articles. These are only examples and the motivation means or motivators associated with each element C can be chosen at will.

Each element C has an associated validation block (labelled as elements VC in Fig. 1, ranging from VC-1 to VC-P), which in this example describes the parameters that are to be monitored to determine whether the user is engaging with this motivation means. Under an element C, all possible materials corresponding to this motivation means are listed together with tags that describe their content. For example, if the element C-3 corresponds to video content, the materials associated with this element are videos and each video includes tags such as sport, competitive, family, emotional or divulgation.

Furthermore, as shown in Fig. 1, the model comprises there are also global elements (elements D, ranging from D-1 to D-L and element E), which control e.g. aesthetic aspects of the graphical user interface, language aspects and usage patterns, to mention a few. Each of the elements D includes a description of how to measure and quantify the monitored aspect. A global element, block E, is the control block. This element is responsible for the actual personalisation (e.g. deciding to switch from video-1 to video-2 to improve the engagement) and communicating to external blocks. This element E has access to all other blocks.

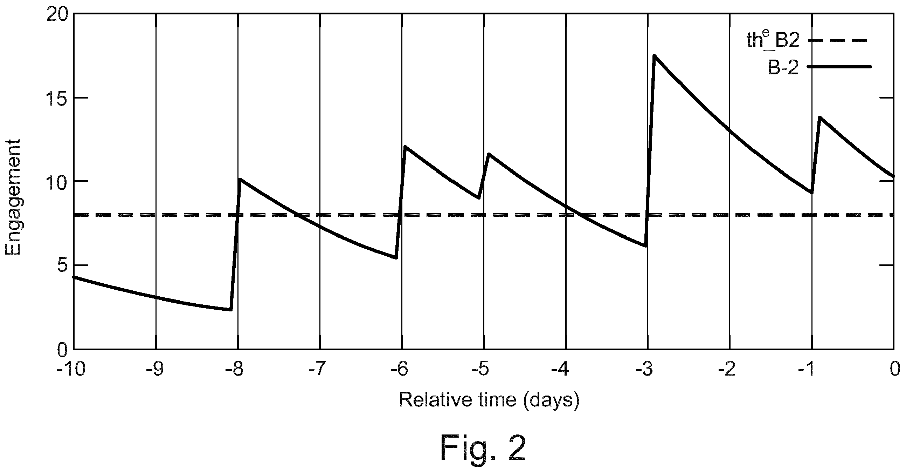

In the following, an exemplary way of measuring user engagement is illustrated referring to Fig. 2. According to an embodiment of the invention, the device is configured to assess the engagement of the user in an activity or in a motivator as described in the following section. For example, the device may be configured to perform a calculation according to one or more of the formulae reproduced in the following disclosure.

The term user engagement in this example preferably only concerns the level of involvement of the user, the predisposition of the user towards carrying out the activities required, and not the completion of the activity or the outcome for the user as a result of engaging in the activity. In other words, a user is highly engaged with an activity at the moment that they systematically perform attempts at completing it (regardless of completing it or not).

For example, if a user is instructed to perform a specific activity for 20 mins every day, a high engagement with this activity is achieved when the user performs the activity every day, at least once a day and spends a minimum of 10 minutes engaging in the activity every day. Systematically attempting but failing to achieve the goal may indicate that the level of the activity is not appropriate for this user, for example the user may not yet competent to perform a given task.

The way of measuring the user engagement depends on the nature of the activity to be carried out. Preferably the mechanism of measuring the user engagement is automatic, without having to explicitly ask the user provide an input or preferably without the user even noticing. For most activities monitoring the number of attempts and the time-length of the attempts (or amount of completion, depending on the activity) already provides valid insights about user engagement.

Gathering insights about user engagement is further described below; leaving this aside for now and assuming that the engagement in each individual attempt can be measured and quantified, the activity engagement $e_B(t)$ (wherein the activity corresponds to an element B in the model as shown in Fig. 1), can be modelled in the following way (see figure 2):

The activity engagement is modelled as a variable, whose minimum value is 0 (no engagement). An activity engagement threshold value $e\_th$ is defined as well; this value must be larger than zero. The higher the value, the higher the engagement.

Each attempt at completing the activity is preferably incorporated to the activity engagement as a function $f(t)$. This function $f(t)$ should be zero before the attempt; steeply increase up to a maximum value at the moment of the attempt and then monotonously decrease in time (towards 0). The term $t$ may be regarded as referring to time, with $t = 0$ referring to the present time. Negative values of t indicate past events and positive values of t indicate future events.

A valid definition is

$$f(t) = Au\left(t + t_0\right)e^{-\frac{t+t_0}{\tau}}$$

where t0 expresses the time elapsed since the attempt took place until now, $\tau$ is a time constant, A the intensity of the attempt (this is further elaborated below) and $u(t)$ is the heavyside function defined as u(t) = 0 if t<0, 1 otherwise.

To combine multiple events, the total engagement $e_B(t)$ may be evaluated as the addition of all individual activity contributions $fi(t)$

$$e_B(t) = \sum_i f_i(t)$$

This function can be used to plot summaries, for reporting purposes. To evaluate the current engagement, it is only necessary to evaluate the current value (this is, t=0).

$$e_B(0) = \sum_i f_i(0)$$

From all past events, only those whose contribution is large enough may be considered, the rest can be neglected. For instance, the contribution must be larger than 0.01$e\_th$; formally $f_i(0) > 0.01e\_th$.

The value of the different parameters must be set accordingly with the goals or instructed activities. A feasible way to determine $\tau$ is to assume high engagement for a long period of time; then $\tau$ must be set so that the engagement is higher than a certain value, e.g. 2$e\_th$.

This can be illustrated with an example: the instructed activity is performing a maintenance work routine three to four times a week that comprises walking 10 min to fetch a component.

Every time that the user starts walking, the contribution is set to *A=1.5*; this value increases the longer the user walks or the more steps the user takes; if walking around 10 min, the contribution is increased to *A=2.5*. The threshold is also *e_th* = 2.5 (exactly the same as the contribution of a single day because it is pursued that the user engages regularly).

In such a case, since the engagement has been high for a long time, there will be a very large number of past events; so large, that the contribution of the oldest will already be below the threshold. Considering all this, defining a $\tau$ of three days considers the events of the (approximately) last two weeks and, if the user regularly walks the required distance, the aggregated contribution is about 2$e\_th$ (see table 1).

The exponential function described above is just an example; different definitions for the function f(t) may be used. A simpler example is f(t) = 1 if t $\in$ [-t0, -t0+$\tau$], 0 otherwise.

In the following an example is given of quantifying the intensity of an attempt of the user to perform an instructed task. In the previous example, the amplitude A was evaluated based on the attempt itself and the time spent walking or the number of steps taken (validation for this activity).

These two validation elements, the attempt itself and the amount of completion or the time spent, are a valid criteria for most activities, but generally these are not representative enough.

For instance, in the case wherein a video is presented to the user via a smartphone the user may leave the phone unattended and do something different. Without any other input, this situation would be considered as high engagement, when it should not be.

Next, examples of objective criteria to measure engagement are listed. These may be valid for both activities (elements B) and motivation means (elements C) as shown in Fig. 1.

For instance, reading could be an instructed activity (corresponding to an element B in Fig. 1, for example reading a chapter of a book) but it can also a motivation means (corresponding to an element C in Fig. 1, like a short text explaining why is it important to perform the instructed activity). The following exemplary criteria may be analysed by the model of a device according to the invention to quantify the intensity of an attempt of the user to perform an instructed task:

The number of attempts. This applies to most activities: physical activity, such as walking or fetching, operating a lever, reading a document, watching a video, etc. This metric corresponds to simple counting of the number of attempts the user makes at completing the instructed activity.

Attempt duration. This applies to most activities: physical activity, reading a document, etc. The amount of time that the user spends trying to complete the activity is measured. An attempt at reading a document (displayed for example by a smartphone or any device with an internal clock) would be quantified by monitoring the amount of time that the document is displayed; similarly for any other material such as audio, video, images, games and interactive inputs.

When a physical activity is instructed (e.g. walking), the amount of time the user spends walking (corresponding to an elapsed time between start of the walk and end of the walk) is monitored. The measured time, e.g. a number in seconds, preferably is be translated into a different value, for example into a score in a range from 0 to 10. This translation can be performed in different ways, for instance, by normalising the measured time by a fixed value which expresses the amount of time that is expected to be used by a user to complete the task. And this value can saturate at a maximum value. Formally, a1 = Δt/(2·T0) if Δt < 2·T0, 1 otherwise; where Δt expresses the measured time and To the expected time required to complete the task.

Active screen watching. When the activity consists in attentively watching something displayed on the screen (images, texts, videos, etc.), it can be verified that the user is looking at the screen the content is output. This requires a camera. The amount of active time watching can be defined as the ratio of the amount of time that the user watches the screen *Δt* compared to the total time where the content is reproduced To; this is especially suited for videos.

$$a_2 = \frac{\Delta t}{T_0}$$

For images and texts, it may be relevant to compute the time that the user actively watches the screen for each image/text fragment *Δt_i*, and normalise this measured value with an expected value for that image/text fragment T0i; and then average to the number of images or fragments N. Further criteria may involve a minimum time per image/fragment, a saturation as in the attempt duration, weighted averages, etc.

$$a_2 = \frac{1}{N} \sum_{i=1}^{N} \frac{\Delta t_i}{T_{0i}}$$

Active screen watching can be evaluated with a face tracker and a camera. Interaction of a user with a device often involves interaction of the user with a smartphone or tablet, which regularly include a front camera. When the user watches the screen, the front camera captures a close-up of the face of the user; the face is then perpendicular to the camera (frontal view). In a first approximation, active screen watching (Δt in the examples above) is detected to occur when the camera captures a front view of the user's face, according to a different embodiment, also the pupil may be tracked. Preferably, the face is identified; this can be done with a face tracker. Face trackers can not only identify the face contour but also landmarks and contours on the face, like the eyes, upper and lower eyebrow bounds, nose bridge, nose bottom or the upper and lower bounds for both the upper and lower lip. With all this information, the orientation of the face can be evaluated with; when the detected orientation is frontal, then the user is actively watching. Otherwise (different orientation, not enough landmarks detected or no face detected at all), the user is not watching the screen.

Active listening. Verifying that a user is listening to an audio is more challenging than verifying active screen watching. A user may play the audio and fall asleep, start talking with another person or watch TV or carefully listen to the audio. A necessary (yet not sufficient) condition for active listening is the amount of background noise. Thus, quantifying background noise may be used to quantify attentive listening. When the audio is played by the speakers, the noise captured by the microphone must not exceed a certain threshold. This threshold may be adapted depending on the play volume and depending on whether headphones are used.

Physical activities can be monitored with the use of sensors, for example sensors present on wearable devices worn by the user. In particular, GPS and pedometer (accelerometer data) can be used to objectively quantify walking activity and distance travelled. Additionally, the heart rate of the user may also be monitored, which can be used to confirm that walking is associated with an increased effort.

Generally, physiological parameters of the user may be monitored, for example the breathing rate and the pulse rate. These parameters may be measured directly or for example extracted from a captured video of the user (e.g. chest movements relating to breathing rate). These physiological parameters may be used by the model to select a suitable motivator, for example a user that has a high pulse rate and breathing rate may respond better to calming motivators and a user that has a low pulse rate and breathing rate may be stirred up by a more stirring motivator. A device according to the present invention may draw on measurements data stemming from sensors present on external devices, for example wearable devices worn by the user or other devices, such as a tablet, a smartphone or a laptop etc. Thus, sensors for monitoring physiological parameters of the user may be present in or at the device itself or may be present on other, external, devices.

When performing activities, the position of the user may change and the user may even lay down. In such a case, the device, for example a smartphone, can be placed on their chest and measure both the pulse rate and the respiration rate from the accelerometer sensor. If the user sits, the heart rate and the respiration rate can be remotely monitored with a camera, e.g. by placing the phone in front of the user pointing towards them.

Regardless of the sensor used, a waveform tracking the user breathing is available. If the waveform relates to the chest volume and not to the change of the chest volume, the breathing stages can be identified by computing the derivative of this raw breathing waveform: inhalations are captured as positive derivative values, exhalations are captured as negative derivative values and apnoeas are captured as very small amplitude derivative values (close to zero). Since breathing can be consciously controlled, the instantaneous breathing waveform may be required; a value expressing the average breathing rate may not be enough.

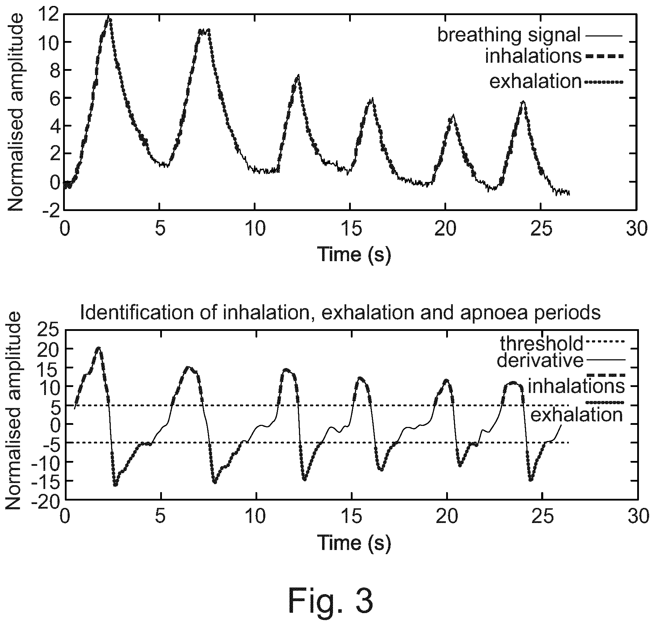

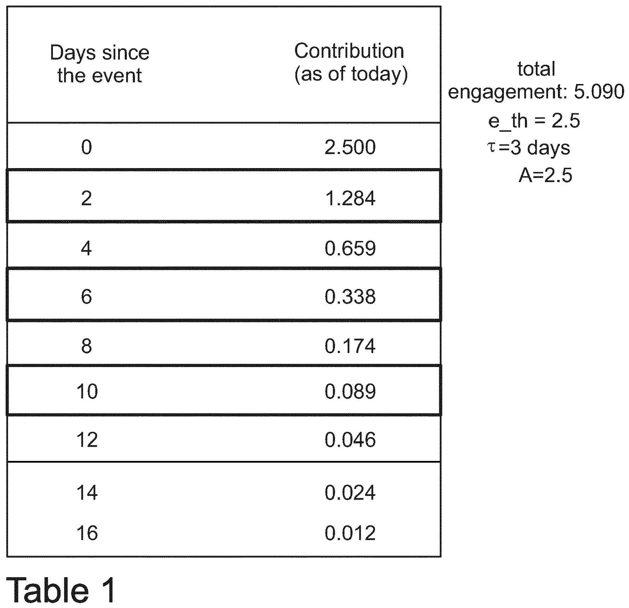

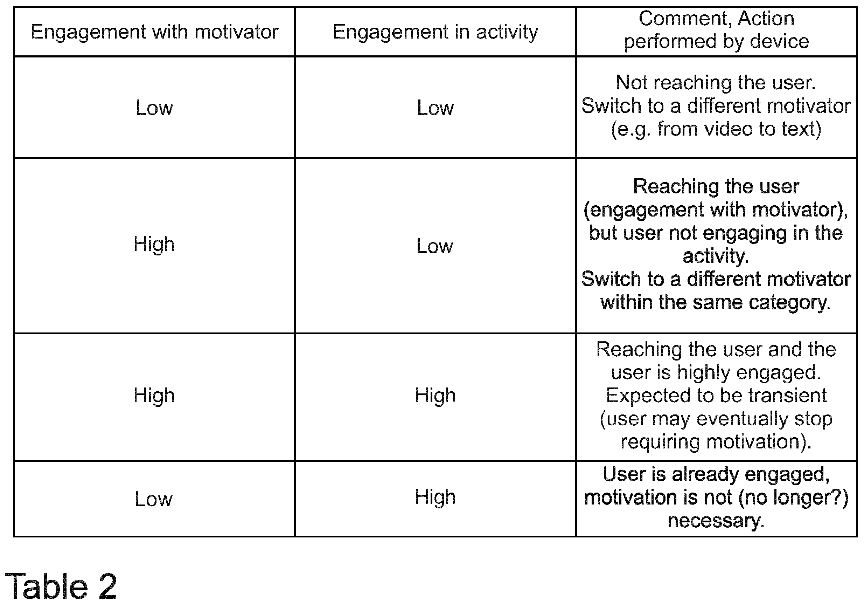

Figure 3 illustrates this with an example. In this case, the breathing waveform is obtained with a camera. The waveform relates to the chest volume (upper plot).

The derivative is evaluated with the kernel [-4, -3, -2, -1, 0, 1, 2, 3, 4]; the derivative signal (very noisy) is smoothed with a median filter and a moving-average filter, both of length 9. The filtered signal (the one displayed in the lower plot of figure 3) is then compared to a threshold, to identify the increasing (inhalations) and decreasing (exhalations) intervals (the segments of the signal belonging to none of these, are then apnoeas). The intrinsic filter delays have been compensated before plotting, for the sake of clarity.

The movement of the heart is not directly controllable by the user. However, if properly engaging in some activities, such as the user standing still to read a text, it is expected that the average heart rate of the user decreases or remains within a certain range of values. Furthermore, certain patterns in the heart rate or the measured waveform may be indicative of a relaxed state, such as Respiratory Sinus Arrhythmia (synchronous variation of the instantaneous heart rate and respiration). All these features can be used as an additional criteria to verify user engagement in an instructed activity.

In the following, an example is given of distinguishing between user preference, e.g. the user subjectively prefers texts, and user needs, e.g. the user objectively needs to be exposed to videos to be motivated to perform an instructed activity. Distinguishing between user preferences (that which is preferred, claimed or consciously chosen by a user) and user need (that which is effective, gets most attention or is mostly used by the user) is an important aspect of this invention. The user preference is a subjective choice and therefore prone to biases (voluntarily or involuntarily). For instance, a user may claim to enjoy reading (user preference), but when prompted with a text, they may quickly skip it and stop at the drawings; therefore, it may be easier reaching to that user by presenting images and videos to them (user need) instead of texts (user preference). Properly distinguishing between user preferences and user needs requires continuous user monitoring whilst the user is interacting with the device and attempting to complete the instructed activity.

User preferences are identified by directly asking the user their opinion or preference on something. User needs are identified by observing the behaviour of the user when prompted with different kinds of inputs, for example by means of a monitoring unit comprising sensors.

The user preference can be used to initialise the model, as a starting point. For example, based on the user stating that they prefer texts, the model initially will select motivators in the form of texts. Relying on the measurements described above, preferably the model is subsequently customized to objectively maximise the engagement of the user in the different activities that the user is instructed to do. Thus, based on the acquired data capturing the behaviour of the user the model may with time change the type of motivator selected, e.g. images instead of texts. In principle, the model could be initialised to any state and, after some interactions with the user, the same end state may be reached. Nevertheless, in practice, it is advisable to start from a known state and, if possible, already close to the final desired state.

As discussed above, two different sorts of engagements are to be distinguished: engaging in the activities themselves (the elements B, the instructed activity, e.g. walking) and engaging with the preparation or the motivation (the elements C, the means to reach B, e.g. the text why it is required to go and fetch a component). The ultimate goal is to get the user engaged with the element B; to reach this goal the elements C are optimized. In this context, preferably, engaging with element B is to be understood as systematically attempting to complete the activity corresponding to element B, but not necessarily actually completing it. Depending on how is the user engaging in these two, different actions may be taken by a device according to the invention. Table 2 shows some examples.

In the following, an example is given of an embodiment of the invention in that the intensity of several attempts of completing an instructed activity is given.

According to this embodiment, the device is preferably configured to generate an attempt intensity score for each attempt a user makes at completing an instructed activity. This allows to quantify how hard the user is trying to complete the instructed activity.

In this example a user is instructed to do a 30 min work routine every day (corresponding to an element B). This user claims to feel comfortable with explanatory texts, so initially they are prompted to perform the routine with texts that describe why the work routine is important (corresponding to an element C). When prompted with the text, the user reads it (active reading) in half the expected time (3min).

$$A1\_C1 = 1{,}3 \text{ min} / 3 \text{ min} = 0{,}43.$$

The intensity of this attempt is low and therefore the engagement in this element C is low (in this example, the engagement in the element C is computed as the average of the last three events). Furthermore, the user does not attempt to complete the work routine but stops after reading the text.

A second day, the user is prompted with a shorter text (2min) which also has an image, but a similar situation happens.

$$A2\_C1 = 1{,}1 \text{ min} / 2 \text{ min} = 0{,}55.$$

However, the user stops at the image and actively watches it for 10s, which is the expected engagement time for this image. Therefore

$$A2'\_C1 = 10s / 10s = 1{,}00.$$

In average, the user is getting an average engagement in the C1 element of 0,49 (low, target is 0,85) and an engagement of 0 in the B element (no attempts). Since both engagements are low, it means that the user is not reached. A different C element has to be explored. The next C element could be chosen at random or, since there is already the insight of the image, the application switches to C2, a video based on scientific analysis.

When prompting the user with that video, the user actively watches the first half, but they fast forward the second half.

$$A3\_C2 = 1,6min / 2min = 0,80.$$

This engagement value was higher than any of the previous C elements, yet not high enough. This suggests that a video is a better way of reaching this user, but the content is not interesting enough to retain the user throughout the whole video. And the user does perform the instructed work routine either.

Next time, a different video is shown. Instead of showing a science-based video, the new video highlights the benefits of performing the work routine, focusing on improvements in the operation of the machine and praise from colleagues. This time, the user actively watches the whole video and attempts to perform the work routine.

$$A4\_C2 = 2min / 2min = 1,00.$$

The average motivation engagement is still low (0,78), yet it is increasing; and so is the activity engagement. Following this approach, the contents that reach the user and result in the user engaging in the B activity can be identified. Note that they do not need to coincide with the conscious user choice (user preference).

Besides the instantaneous engagement, it is also relevant to look at the change in engagement. When, after a period of time of engagement improvements in a certain B activity, a saturation period is reached, it can be notified to a higher module that it may be advisable to change the instructed activity.

Thus, according to an embodiment, the device is preferably configured to detect changes in the attempt intensity score over at least one time interval. The time interval can for example span two attempts so that the change in the attempt intensity score may indicate that the attempts of the user to complete an activity are getting more or less intense.

Besides optimising the motivator(s) used, such as the elements C, to make the user engage with the element B, such as an activity, there are other engagement-related aspects that can be optimised. These refer to the way of interacting with the device itself or an application running thereon. Multiple aspects can be optimised; some of them are listed below.

Furthermore, the way in which the user interacts with the application is something that can be monitored as well, as input for a further customisation.

According to an embodiment of the invention, the device is further configured to analyse the interaction of the user with the device, for example typing patterns of the user on a screen of the device, and preferably infer characteristics of the user therefrom. The characteristics of the user inferred in this way may be entered into the model.

For example, the device may switch from interacting with the user in writing to a voice-based communication, if the analysis has yielded that the user can not easily read and / or write text. In this case, the device may use a speech-to-text conversion unit and / or a text-to-speech conversion unit to interact with the user.

One aspect that can be optimized is the font size and thus accessibility of information presented to the user. For example, displayed elements may be too small for a user to see them. Users may have different visual acuity but also displays of different sizes. Especially when smart phones are used, which have relatively small displays, it is common for users to zoom in to better see details. The detection of frequent zooming in or multiple missed taps around a target point may suggest that the current font size is too small for the user. Thus, if such a detection is made, the control unit may control the device to increase font and button sizes and / or use a high-contrast theme for the graphical user interface presented to the user.

Furthermore, it is possible to determine how much the user relies on examples and additional information; for example by tracking how much the "learn more" (or similar) button is clicked by the user. This provides insights about how much information should be presented to the user at once. Some users may prefer to read first a comprehensive description before performing an activity, some other users may prefer to start with almost no background information and progressively learn by interacting with built-in examples. These insights can be used to adjust the amount of information presented for new activities, in order to make it easier for the user to engage with the device to complete these activities.

Typing patterns of the user may also be monitored. Slow typing, possibly combined with frequently making mistakes when typing (frequent use of the backspace), may be an indication of difficulties in typing. If this is detected, the device may offer enabling speech-to-text support to allow the user dictating instead of typing. When audio-based interactions are offered, a digital assistant may be offered for voice-based interactions between the user and the device.

As part of the global tuning, notifications may also be customised. In particular, reminders may be sent to the user if there were no interactions with the device or application for a certain time period. These notifications may be sent during the time intervals in that the user normally mostly interacts with the application, for example between 8 am and 10 am of every day. According to an embodiment, it can also be monitored whether the user reacts to notifications or chooses to ignore them.

When the user inputs textual information, besides analysing the typing patterns, the text itself may be analysed. Machine learning-based algorithms can be used to infer certain characteristics (e.g. age estimation, education level etc.), even though simpler rule-based algorithms can be used as well. For instance, aspects that can be identified are the time of communication (part of the day, day of the week, etc.), the communication length (short or long messages), the frequency of the communication (e.g. number of times per week) and the vocabulary (technical or general, using abbreviations, slang words, spelling mistakes, etc.). These, however, are only examples.

The model described in figure 1 is optimised towards finding the most effective way to reach the user, in other words, how to approach the user to maximise their engagement in different activities. By combining all engagement information from all blocks, it is possible to infer a general user profile (also referred to as a profile summary) that can be subsequently used to customise a chat bot (also referred to as a virtual support system).

In other words, a device according to the invention may be configured to generate a user profile based on acquired data and / or parameters relating to the user, preferably using the model.

The user profile can be a list of words that characterise that user, or a list of words+score indicating the representativity of that word for that user. It can be created from the engagement values from the engagement validation blocks VB and VC. For instance, by simply creating a number of lists with all tags (from all blocks) and adding the engagement value of each element to the corresponding tags, the predominant tags for that user can be identified. These tags conform (a first version of) the profile summary. Plausibility checks may be added to ensure representativity of the model (such as a minimum number of model updates before providing a first profile summary, or a minimum distance between the dominant tags and the other tags, or normalising the values to a defined scale). The user profile may be enriched with the insights gathered from the global tuning (e.g. if identifying interest in additional information and "learn more", an associated tag could be "curious").

From the list of words describing the user (which, again, has preferably been determined from those aspects such as motivators that are most effective for that user and not from what the user claims), a virtual support system can be tuned accordingly. Not only the aspect (e.g. the appearance of the avatar and background of the avatar etc.) but also the communication style, e.g. formal or colloquial communication, may be tailored to the needs of an individual user.

For instance, from the detected language patterns used by the user, the chat bot may be adapted to write in the same way e.g. using the same kind of abbreviations that the user employs.

Initially this model, e.g. the model shown in Fig. 1, is empty and needs to be initialised. Some guidelines must be provided. These guidelines may be provided by the user directly (e.g. the user entering user preference), by another person, fetched from an external resource (like an electronic record or guideline), initialised by default to a value or inferred from an onboarding test period.

Regardless of the initial state, frequent interactions of the user with the device or application resulting in frequent model updates will ultimately lead to the same result, as the model will over time learn to identify the characteristics of the user and be increasingly capable of distinguishing subjective user preferences from objective user needs. However, it is advisable to initialize the model in a known state or in a state where the user would already feel comfortable with, e.g. a state in that the settings reflect the user preference.

The person skilled in the art will understand that when in this disclosure alternative wording is used to describe the same or similar subject-matter, the alternative terms or words may be used interchangeably or as synonyms.

Similarly, if certain elements are disclosed in singular form, e.g. preceded by the articles "a" or "the", the disclosure is not limited to the singular form but can also contain the element in plural form. The articles "a" or "the" are thus preferably to be understood as "at least a" or "at least the". Conversely, if certain elements are disclosed in plural form the present invention may also contain these elements in singular form.

Furthermore, it is apparent to the person skilled in the art that if certain elements or features of the invention have been disclosed in a certain combination or in the context of a certain embodiment, these elements or features of the invention may also be claimed in isolation or in different combinations or in the context of a different embodiment.

**Claims**

1.  Device for assessing the engagement of a user in at least one activity, wherein the device is configured to:

    - Instruct the user to perform at least one activity by means of an output unit,
    - Monitor at least one first parameter by means of a monitoring unit to assess the engagement of the user in performing the at least one activity or attempting to perform the at least one activity,
    - Expose the user to at least one first motivator by means of the output unit, wherein the motivator is chosen to prompt or motivate the user to perform the at least one activity or to attempt to perform the at least one activity,
    - Monitor at least one second parameter by means of the monitoring unit to assess the engagement of the user in the at least one motivator,

- Enter the first and second parameters into a model of the user that indicates the efficacy of at least one motivator for motivating the user to perform or to attempt to perform at least one activity, wherein the model is preferably customized to the individual user,
- Compare by means of a comparison unit the at least one first parameter or an activity engagement score based on the at least one first parameter to a threshold value, and / or
- Compare by means of the comparison unit the at least one second parameter or an motivator engagement score based on the at least one second parameter to a threshold value,
- If the comparison indicates that the at least one first parameter or the activity engagement score and / or the at least one second parameter or the motivator engagement score is below the threshold value,
- Select by means of the model at least one second motivator from a list of motivators accessible by the model, and
- By means of a control unit control the device to expose the user to the least one second motivator by means of the output unit.

2. Device according to claim 1, wherein the model is continuously updated based on parameters monitored by the monitoring unit, such as the first and second parameters entered into the model, and / or calculated values derived from these parameters.

3. Device according to claim 1 or 2, further configured to:
by means of an evaluation unit evaluate for a past time interval a change in the at least one first parameter or the activity engagement score and / or the at least one second parameter or the motivator engagement score.

4. Device according to one of the preceding claims, configured to:
based on parameters monitored by the monitoring unit, such as the first and second parameters entered into the model, and / or calculated values derived from these parameters, identify by means of the model at least one attribute of the user that characterizes the user and in particular indicates a preference of the user for at least one motivator.

5. Device according to claim 4, configured to:
by means of the model, select from a stored list of support sources based on the at least one attribute of the user a support source expected to optimally support the user in performing the at least one activity or attempting to perform the at least one activity, wherein the support source preferably is person or a virtual support system, such as a chatbot or an avatar.

6. Device according to claim 4 or 5, configured to:
generate based on the at least one attribute of the user a virtual support system expected to optimally support the user in performing the at least one activity or attempting to perform the at least one activity, wherein the virtual support system is a chatbot or an avatar.

7. Device according to one of the preceding claims, configured to:

instruct the user to perform a plurality of activities by means of the output unit, and
for each activity of the plurality of activities expose the user to at least one first motivator, wherein the motivators may differ from each other.

8. Device according to one of the preceding claims, wherein different motivators are configured to convey the same content to the user in different formats and / or modes of delivery.

9. Method for assessing the engagement of a user in at least one activity, the method preferably being performed by a device according to one of claims 1-8, the method comprising:

- Instructing the user to perform at least one activity, preferably by means of an output unit,
- Monitoring at least one first parameter by means of a monitoring unit to assess the engagement of the user in performing the at least one activity or attempting to perform the at least one activity,
- Exposing the user to at least one first motivator, preferably by means of the output unit, wherein the motivator is chosen to prompt or motivate the user to perform the at least one activity or to attempt to perform the at least one activity,
- Monitoring at least one second parameter, preferably by means of the monitoring unit, to assess the engagement of the user in the at least one motivator,
- Entering the first and second parameters into a model of the user that indicates the efficacy of at least one motivator for motivating the user to perform or to attempt to perform at least one activity, wherein the model is preferably customized to the individual user,
- Comparing, preferably by means of a comparison unit, the at least one first parameter or an activity engagement score based on the at least one first parameter to a threshold value, and / or

- Comparing, preferably by means of the comparison unit, the at least one second parameter or an motivator engagement score based on the at least one second parameter to a threshold value,
- If the comparison indicates that the at least one first parameter or the activity engagement score and / or the at least one second parameter or the motivator engagement score is below the threshold value, selecting by means of the model at least one second motivator from a list of motivators accessible by the model, and
- Exposing the user, preferably means of a control unit controlling the device, to the least one second motivator, preferably by means of the output unit.

10. Method according to claim 9, wherein the model is continuously updated based on parameters monitored by the monitoring unit, such as the first and second parameters entered into the model, and / or calculated values derived from these parameters.

11. Method according to claim 9 or 10, further comprising:

    evaluating for a past time interval, preferably by means of an evaluation unit, a change in the at least one first parameter or the activity engagement score and / or
    the at least one second parameter or the motivator engagement score.

12. Method according to one of claims 9-11, further comprising:
    based on parameters monitored preferably by the monitoring unit, such as the first and second parameters entered into the model, and / or calculated values derived from these parameters, identifying by means of the model at least one attribute of the user that characterizes the user and in particular indicates a preference of the user for at least one motivator.

13. Method according to one of claims 9-12, further comprising:
    by means of the model, selecting from a stored list of support sources based on the at least one attribute of the user a support source expected to optimally support the user in performing the at least one activity or attempting to perform the at least one activity, wherein the support source preferably is person or a virtual support system, such as a chatbot or an avatar.

14. Method according to one of claims 12 and 13, further comprising:
    generating based on the at least one attribute of the user a virtual support system expected to optimally support the user in performing the at least one activity or attempting to perform the at least one activity, wherein the virtual support system is a chatbot or an avatar.

15. Method according to one of claims 9 to 14, further comprising:

    instructing the user to perform a plurality of activities by means of the output unit, and
    for each activity of the plurality of activities exposing the user to at least one first motivator, wherein the motivators may differ from each other.

16. Method according to one of claims 9 to 15, wherein different motivators are configured to convey the same content to the user in different formats and / or modes of delivery.

EP 4 246 394 A1

| A-1 | | ... | A-N | |
|-----|-----|-----|-----|-----|
| manufacturing | | | maintenance | |

| B-1 | VB-1 engagement validation | ... | B-M * | VB-M engagement validation |
|-----|-----|-----|-----|-----|
| Read text for 2 min | | | Walk for 3 min | |

| C-1 | C-2 | C-3 | C-4 | C-5 | C-P |
|-----|-----|-----|-----|-----|-----|
| quiz-like games | audio | video | images | interactive inputs | text and articles |
| VC-1 engagement validation | VC-2 engagement validation | VC-3 engagement validation | VC-4 engagement validation | VC-5 engagement validation | VC-P engagement validation |

─video-1: competitive, sport
─video-2: growth, competitive
─video-3: emotional, family
─video-4: divulgative, scientific
⋮

| D1 aesthetics |
|-----|
| D2 communication aspects |
| D3 usage patterns |

⋮

| DL digital assistant |
|-----|
| E global control |

## Fig. 1

Fig. 2

Fig. 3

| Days since the event | Contribution (as of today) |
|:---:|:---:|
| 0 | 2.500 |
| 2 | 1.284 |
| 4 | 0.659 |
| 6 | 0.338 |
| 8 | 0.174 |
| 10 | 0.089 |
| 12 | 0.046 |
| 14 | 0.024 |
| 16 | 0.012 |

total engagement: 5.090

$e\_th = 2.5$

$\tau = 3$ days

$A = 2.5$

# Table 1

| Engagement with motivator | Engagement in activity | Comment, Action performed by device |
| --- | --- | --- |
| Low | Low | Not reaching the user. Switch to a different motivator (e.g. from video to text) |
| High | Low | Reaching the user (engagement with motivator), but user not engaging in the activity. Switch to a different motivator within the same category. |
| High | High | Reaching the user and the user is highly engaged. Expected to be transient (user may eventually stop requiring motivation). |
| Low | High | User is already engaged, motivation is not (no longer?) necessary. |

Table 2

EP 4 246 394 A1

Europäisches
Patentamt

European
Patent Office

Office européen
des brevets

# EUROPEAN SEARCH REPORT

Application Number

EP 22 16 1968

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "FORERUNNER 935 Owner's Manual", , 30 June 2020 (2020-06-30), pages 1-46, XP055953951, Retrieved from the Internet: URL:https://www8.garmin.com/manuals/webhelp/forerunner935/EN-US/Forerunner_935_OM_EN-US.pdf [retrieved on 2022-08-23] * pages 1-7 * * page 16 * * page 25 * ----- | 1-16 | INV. G06Q10/06 G09B19/00 G09B5/06 ADD. G06Q50/04 G09B21/00 |
| X | US 2019/251616 A1 (YANKOVICH STEVE [US] ET AL) 15 August 2019 (2019-08-15) * paragraphs [0033] - [0038]; figure 1 * * paragraphs [0054] - [0057]; figure 3 * ----- | 1-16 | |
| X | WO 2020/257354 A1 (GIDEON HEALTH [US]) 24 December 2020 (2020-12-24) * paragraphs [0029] - [0035]; figure 1 * * paragraphs [0068] - [0073] * ----- | 1-16 | TECHNICAL FIELDS SEARCHED (IPC) G06Q G09B |
| X | US 2019/258944 A1 (LEE MATTHEW [US] ET AL) 22 August 2019 (2019-08-22) * paragraph [0001] * * paragraphs [0038] - [0049]; figures 1,3 * ----- | 1-16 | |
| X | US 2016/005320 A1 (DECHARMS CHRISTOPHER [US] ET AL) 7 January 2016 (2016-01-07) * paragraphs [0290] - [0292] * * paragraph [0420] * * paragraphs [0059] - [0069]; figure 1 * ----- | 1-16 | |

-/--

The present search report has been drawn up for all claims

| Place of search | Date of completion of the search | Examiner |
|---|---|---|
| Munich | 24 August 2022 | Giemsa, Falk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

# EUROPEAN SEARCH REPORT

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 518 055 B1 (IGZ INGENIEURGESELLSCHAFT FUER LOGISTISCHE INFORMATIONSSYSTEME MBH [DE] 29 September 2021 (2021-09-29) * paragraphs [0010] - [0012] * * paragraph [0034] * * paragraphs [0045] - [0052]; figure 1 * ----- | 1-16 | |
| | | | TECHNICAL FIELDS SEARCHED (IPC) |

The present search report has been drawn up for all claims

| Place of search | Date of completion of the search | Examiner |
|---|---|---|
| Munich | 24 August 2022 | Giemsa, Falk |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.............................................................................................
& : member of the same patent family, corresponding document

EPO FORM 1503 03.82 (P04C01)

EP 4 246 394 A1

## ANNEX TO THE EUROPEAN SEARCH REPORT
## ON EUROPEAN PATENT APPLICATION NO.

EP 22 16 1968

24-08-2022

| Patent document cited in search report | | Publication date | Patent family member(s) | | Publication date |
|---|---|---|---|---|---|
| US 2019251616 | A1 | 15-08-2019 | CN | 111712846 A | 25-09-2020 |
| | | | CN | 111712847 A | 25-09-2020 |
| | | | US | 2019251613 A1 | 15-08-2019 |
| | | | US | 2019251616 A1 | 15-08-2019 |
| | | | US | 2019253514 A1 | 15-08-2019 |
| | | | US | 2020404072 A1 | 24-12-2020 |
| | | | US | 2021368021 A1 | 25-11-2021 |
| | | | WO | 2019160601 A1 | 22-08-2019 |
| | | | WO | 2019160602 A1 | 22-08-2019 |
| | | | WO | 2019160603 A1 | 22-08-2019 |
| WO 2020257354 | A1 | 24-12-2020 | NONE | | |
| US 2019258944 | A1 | 22-08-2019 | JP | 2019145067 A | 29-08-2019 |
| | | | US | 2019258944 A1 | 22-08-2019 |
| US 2016005320 | A1 | 07-01-2016 | US | 2016005320 A1 | 07-01-2016 |
| | | | US | 2016267809 A1 | 15-09-2016 |
| | | | WO | 2016004396 A1 | 07-01-2016 |
| EP 3518055 | B1 | 29-09-2021 | DE | 102018000733 A1 | 01-08-2019 |
| | | | EP | 3518055 A1 | 31-07-2019 |
| | | | EP | 3929675 A1 | 29-12-2021 |

EPO FORM P0459